# EUROPEAN PATENT SPECIFICATION

(11) **EP 1 582 227 B1**
(45) Date of publication and mention of the grant of the patent: **19.11.2008**
(21) Application number: 05012011.2
(22) Date of filing: 07.07.2003
(51) Int. Cl.: A61M 1/28

(54) **Peritoneal dialysis apparatus**
Gerät zur Peritonealdialyse
Appareil de dialyse péritonéale

(30) Priority: 19.07.2002 JP 2002210325; 25.07.2002 JP 2002216557; 29.07.2002 JP 2002219138
(43) Date of publication of application: 05.10.2005
(62) Divisional of application: 03254309.2
(73) Proprietor: TERUMO KABUSHIKI KAISHA, Tokyo (JP)
(72) Inventor: Suzuki, Minori, Fijinomiya-shi Shizuoka-ken, 418-0004 (JP); Suzuki, Hironobu, Fijinomiya-shi Shizuoka-ken, 418-0004 (JP)
(74) Representative: Paget, Hugh Charles Edward

(56) References cited:
- EP-A- 1 195 171
- US-A- 5 141 492
- US-A- 5 438 510
- US-A- 5 445 610
- US-A- 6 117 122

## Description

The present invention relates to a peritoneal dialysis apparatus usable by a patient at home.

A continuous ambulatory peritoneal dialysis (hereinafter, referred to as "CAPD") has been a focus of great attention because of its advantage in allowing a patient to exchange a dialysis fluid container (bag) at home or office, thereby promoting the patient's social rehabilitation.

The CAPD involves implanting a catheter tube (peritoneal catheter) in a peritoneal cavity of a patient, connecting a transfer tube to the end, located outside the patient's body, of the catheter tube, connecting a bag tube of a dialysis fluid bag (fluid infusion bag), infusing a dialysis fluid in the bag in the peritoneal cavity through each tube, and recovering the drained fluid in the peritoneal cavity in the drained fluid bag through each tube. In addition, the connection between the tubes is aseptically performed by fitting male connectors and female connectors mounted to the ends of both the tubes.

By the way, in the CAPD, the dialysis fluid is infused to the inside of the peritoneum by placing the dialysis fluid bag at a position higher than the patient's abdomen by about 1 m and transferring the dialysis fluid from the dialysis fluid bag into the patient's peritoneal cavity under the gravity. The spent dialysis fluid from the patient's peritoneal cavity is recovered by placing the drained fluid bag at a position lower than the patient's abdomen by about 1 m and transferring the dialysis fluid from the inside of the patient's peritoneal cavity to the drained fluid bag under the gravity.

According to this method of infusing and draining a dialysis fluid, in the case of performing peritoneal dialysis for a sleeping patient, the patient must be laid at a position higher than the floor by about 70 cm to 100 cm by using a bed, and the dialysis fluid bag must be set at a position higher than the patient by about 1 m. As a result, the height of the entire apparatus becomes as large as about 2 m. Such an apparatus is difficult to handle and transport. Moreover, if the patient turns over during sleeping, the apparatus might fall. Since gravity necessary for draining the fluid must be maintained, the patient's sleeping position (height) cannot be freely selected.

To solve these disadvantages, there has been proposed a peritoneal dialysis apparatus intended to automate fluid infusion and fluid drainage and to eliminate the limitation in heights of the location on which a dialysis fluid bag and a drained fluid bag are placed. For example, Japanese Patent No. 3113887 has proposed a peritoneal dialysis apparatus in which the flow path of the disposable cassette is switched by opening/closing a valve with a valve actuator. Japanese Patent Laid-Open No. Hei 11-347115 has proposed a disposable cassette integrally having a heating portion and a pump (diaphragm) for delivering a peritoneal-dialysis fluid. This cassette is heated from both the sides, and the heated peritoneal dialysis fluid is delivered into the patient's peritoneal cavity with two pumps (diaphragms).

However, to perform peritoneal dialysis at home by using such a peritoneal dialysis apparatus, the patient must receive sufficient training for making use of the peritoneal dialysis apparatus, store all the procedures, and operate the apparatus correctly. This is no small burden to the patient.

The related art peritoneal dialysis apparatus also has an inconvenience that since a patient cannot sleep before the operation of the apparatus is started, if the patient is intended to start therapy in the night (late at night or before dawn), the patient must keep awake until that time.

A further problem of the related art peritoneal dialysis apparatus is that even if a patient encounters a slight trouble related to the operational procedure, the patient cannot cope with the trouble quickly. In the related art peritoneal dialysis apparatus, when peritoneal dialysis is performed during sleeping, if tubes of a dialysis circuit are closed (occluded) by turn-over of a patient, it may fail to continue fluid infusion or fluid drainage at a specific fluid infusion rate or fluid drainage rate, to generate an alarm indicating the defective fluid infusion or fluid drainage. However, when the patient is turn-over again, the closure (occlusion) of the tubes may be released, to cause a possibility that the fluid infusion rate or fluid drainage rate is returned to an normal rate. Accordingly, the generation of alarm for each temporary closure of tubes may obstruct the sleeping of the patient.

The related art peritoneal dialysis apparatus has a further inconvenience that the dialysis fluid must be drained from a peritoneal cavity by temporarily stopping the staying operation in order for a patient to go out for 1 to 2 hr during therapy or to do household affairs for about several tens hr during therapy.

A preferred aim of the present invention is to provide a peritoneal dialysis apparatus capable of automating self-dialysis therapy by a patient, significantly simplifying the operation of the apparatus, and setting a dialysis end time even in either a first therapy mode (or so-called A mode) adapted to calculate a total amount of dialysis fluid on the basis of inputted set-up parameters; an amount of infusion fluid, a staying time(a staying duration), and the number cycles or a second therapy mode (or so-called B mode) adapted to calculate a total amount of dialysis fluid and a staying time on the basis of inputted set-up parameters: a dialysis time(a dialysis duration), an amount of infusion fluid, and the number of cycles, thereby performing the dialysis therapy in optimal conditions.

Another aim of the present invention is to provide a peritoneal dialysis apparatus capable of automating self-dialysis therapy by a patient, significantly simplifying the operation of the therapy, and eliminating generation of an unnecessary alarm even if fluid infusion or fluid drainage cannot be performed at a specific fluid infusion rate or fluid drainage rate due to temporary closure of tubes caused by turn-over of the patient, thereby performing dialysis therapy in optimal conditions.

A further aim of the present invention is to provide a peritoneal dialysis apparatus capable of automating self-dialysis therapy by a patient, significantly simplifying the operation of the apparatus, and eliminating the need of temporarily stopping a staying operation for fluid drainage, and allowing the patient to go out for a staying time of about 1 to 2 hr, thereby performing dialysis therapy in optimal conditions.

EP-A-1195171 discloses the compact peritoneal dialysis apparatus using a disposable cassette, which is the same as the apparatus described below by way of example, and illustrated by the drawings accompanying the present specification, to which the present invention is applied.

US 5141492 describes a peritoneal dialysis apparatus, having a safety provision to determine whether a sufficient quantity of dialysate has drained from the patient's cavity during the drain phase. If not, the apparatus is shut down.

According to the present invention, there is provided a peritoneal dialysis apparatus as set out in claim 1. The fluid infusion time (min) may be obtained by adding a preparation time until the start of fluid infusion to a value of the amount of infusion fluid (mL)/the fluid infusion rate (mL/min).

### BRIEF DESCRIPTION OF THE DRAWINGS

These and other objects, features and advantages of the present invention will become more apparent from the following description taken in conjunction with the accompanying drawings wherein:
FIG. 1 is a perspective view showing the appearance of a peritoneal dialysis apparatus with a cassette disclosed in EP 1195171, which may be used in the present invention;
FIG. 2 is a typical diagram of an example of a peritoneal dialysis apparatus disclosed in EP 1195171 which is usable in the present invention;
FIG. 3 is a perspective view showing a flow passage switching portion and clampers of the cassette;
FIG. 4 is a three-dimensional exploded view of the cassette;
FIG. 5 is a view showing a relationship between a heating circuit of the cassette and heaters;
FIG. 6A is a typical view showing a state that a dialysis fluid is delivered in a peritoneal cavity, and FIG. 6B is a typical view showing a state that a drained fluid is delivered;
FIG. 7 is a block diagram of a dialysis apparatus body;
FIGS. 8 to 8C are diagrams showing display screens sequentially variable and display screens for patterns of therapy conditions in a displaying portion;
FIG. 9 is a diagram showing display screens sequentially variable on the displaying unit;
FIG. 10 is a diagram showing display screens sequentially variable on the displaying unit;
FIG. 11 is a diagram showing display screens sequentially variable on the displaying unit;
FIG. 12 is a diagram showing display screens sequentially variable on the displaying unit;
FIG. 13 is a diagram showing display screens sequentially variable on the displaying unit;
FIG. 14 is a flow chart for detecting defective fluid infusion and defective fluid drainage; and
FIGS. 15A to 15C are diagrams showing screens displaying temporary separation.

### DETAILED DESCRIPTION OF THE PREFERRED EMBODIMENT

A peritoneal dialysis apparatus of the present invention will be described in detail by way of a preferred embodiment shown in the accompanying drawings.

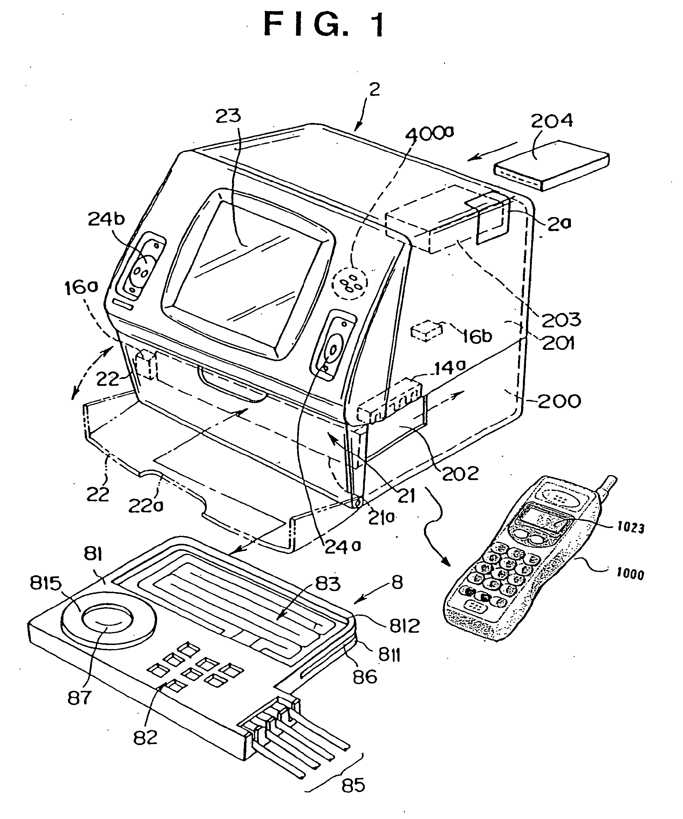

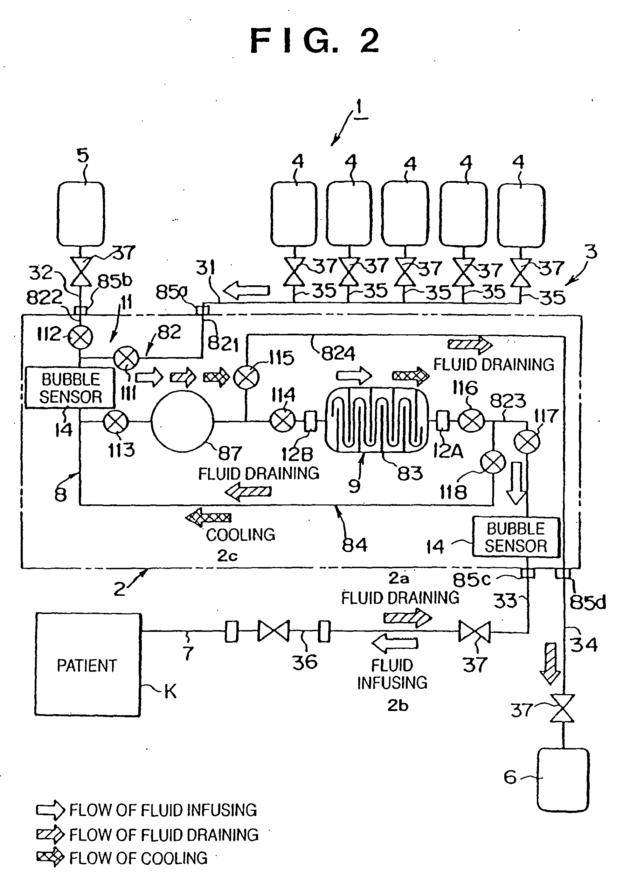

FIG. 1 is a perspective view showing the appearance of the peritoneal dialysis apparatus with a disposable cassette (peritoneal dialysis circuit) 8 (disclosed in EP 11951171) to which the present invention is applied, and FIG. 2 is a typical diagram showing the entire configuration of the peritoneal dialysis apparatus of the present invention.

Referring to FIGS. 1 and 2, there is shown a peritoneal dialysis apparatus 1 including a dialysis apparatus body 2 and a cassette 8 for the peritoneal dialysis apparatus. The cassette 8 is removably mounted on the dialysis apparatus body 2.

As shown in FIG. 1, the dialysis apparatus body 2 includes a cassette mounting portion 21 having an opening portion 21a indicated by a two-dot chain line, a lid member 22 having a holding portion 22a, a display portion 23, an operating portion (start switch) 24a for performing an operation of starting therapy, and an operating portion 24b (stop switch) for performing an operation of stopping therapy. The cassette 8 is mounted from the front side to the cassette mounting portion 21 through the opening 21a. The lid member 22 is operated by a user holding the holding portion 22a in such a manner as to be turned between a close position indicated by a solid line at which the lid member 22 closes the cassette mounting portion 21 and an open position indicated by a broken line at which the lid member 22 opens the cassette mounting portion 21.

The operating portions 24a and 24b are different from each other in shape and color for easy discrimination. For further discrimination, the operating portion 24a for starting, that is, serving as the start switch has one projection, whereas the operating portion 24b for stopping, that is, serving as the stop switch has two projections.

The display portion 23 is typically configured as a touch-sensitive panel portion including a liquid crystal display (LCD) panel or the like. In accordance with touch operation of the display portion 23, the display portion 23 displays various kinds of information necessary for dialysis, and also displays how to operate the apparatus together with a voice guide given from a speaker 400a indicated by a broken line, to thereby ensure operability and convenience of the apparatus.

A sensor 16a, a sensor 16b, and a bubble sensor 14a are disposed at positions shown in FIG. 1. The sensor 16a detects that the lid member 22 is in the closed state indicated by the solid line. The sensor 16b detects that the cassette 8 has been loaded. The bubble sensor 14a detects that bubbles has been entrained in connection tubes 85 connected to the cassette 8.

A hook 2a is provided on a cover portion of the dialysis apparatus body 2 so as to be storable in the cover portion. The tubes are hung from the hook 2a, to thereby ensure the delivery of a dialysis fluid.

The dialysis apparatus body 2 has a mounting base composed of a main base 200 and a sub-base 201 indicated by broken lines, and resin covers provided for the main base 200 and the sub-base 201. Each of the main base 200 and sub-base 201 is formed of an aluminum plate having a thickness of 1 mm to 2 mm, and has large holes at several portions, thus reducing the weight. The lightweight resin covers are fixed to the main base 200 and the sub-base 201. A memory card 204 having a memory capacity of, for example, 100 megabytes or more is loadable in a card reader 203 (indicated by a broken line) from the back surface of the apparatus, to thereby quickly change the display content of the display portion 23, the voice guide, and specifications for different countries. In addition, by storing patient data in the memory card 204, the apparatus can respond to individual patients.

A shield plate 202 is provided to the right surface of the cassette mounting portion 21 indicated by a two-dot chain line. The shield plate 202 is movable in the direction of an arrow indicated by a broken line, to prevent mechanical interference between the shield plate 202 and the connection tubes 85 of the cassette 8, thereby allowing the cassette 8 to be set at a loading position without such mechanical interference.

The cassette 8 is composed of a cassette body 81, a lower body frame 811, and an upper body frame 812. The cassette body 81 has a shape allowing the cassette body 81 to be removably mounted on the cassette mounting portion 21 of the dialysis apparatus body 2. The lower body frame 811 is formed so as to be continuous to the cassette body 81. The upper body frame 812 is provided so as to be opposed to the body frame 811 with a gap 86 put therebetween.

The cassette body 81 integrally has, as shown in FIG. 1, a fluid delivery diaphragm 87, a heating portion 83, and a flow passage switching portion. The periphery of the diaphragm 87 is surrounded by a flange member 815.

Referring to FIG. 2, the peritoneal dialysis apparatus 1 includes a dialysis fluid circuit unit 3. The dialysis fluid circuit unit 3 is connected to a plurality of dialysis fluid bags (dialysis fluid containers) 4, an additional dialysis fluid bag 5, a drained fluid tank (drained fluid container) 6, and a dialysis catheter (catheter tube) 7. The dialysis fluid bags 4 contain a dialysis fluid to be infused (delivered) in a peritoneum (peritoneal cavity) of a patient K. The additional dialysis fluid bag 5 contains a dialysis fluid with a different concentration. The drained fluid tank 6 recovers the dialysis fluid drained from the inside of the peritoneum of the patient K. The dialysis catheter 7 is implanted inside the peritoneum of the patient K.

The dialysis fluid circuit unit 3 has a fluid infusion tube circuit (line) 31, an additional fluid infusion tube circuit (line) 32, a fluid infusion/drainage tube circuit (line) 33, and a fluid drainage tube circuit (line) 34. The dialysis fluid circuit unit 3 also has a switching cassette circuit 82, a heating cassette circuit 83, and a bypass circuit (patient-side tube circuit) 84, each of which is provided on the cassette body 81 of the cassette 8. The switching cassette circuit 82 is composed of a fluid infusion circuit 821, an additional fluid infusion circuit 822, a fluid infusion/drainage circuit 823, and a fluid drainage circuit 824.

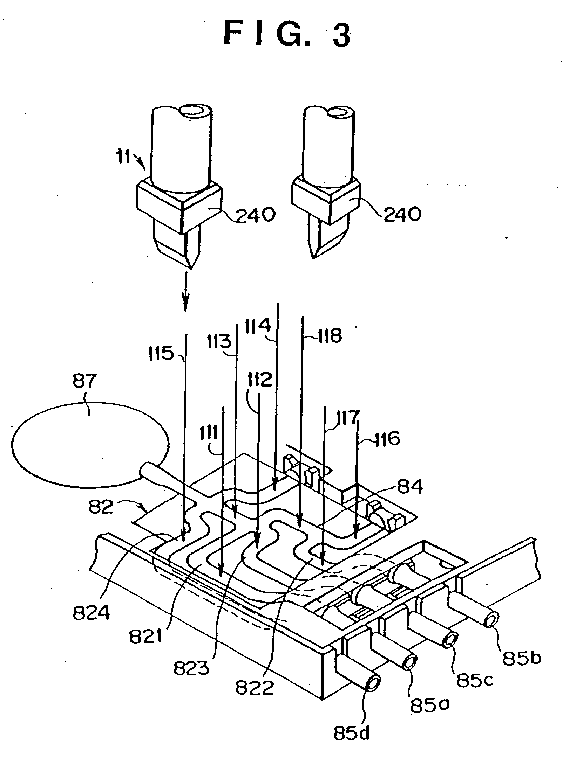

FIG. 3 is a perspective view showing the appearances of the flow passage switching portion of the cassette 8 and clampers (clamps) 240. One end of the fluid infusion circuit 821, one end of the additional fluid infusion circuit 822, the other end of the fluid infusion/drainage circuit 823, and the other end of the fluid drainage circuit 824 are connected to connection tubes 85a, 85b, 85c, and 85d, respectively.

Referring again to FIG. 2, one end of the fluid infusion tube circuit 31 is branched to a plurality of branch tube circuits 35. One end of each branch tube circuit 35 is connected to the corresponding one of the plurality of dialysis fluid bags 4. The other end of the fluid infusion tube circuit 31 is connected to one end of the fluid infusion circuit 821 through the connection tube 85a.

One end of the additional fluid infusion tube circuit 32 is connected to the additional dialysis fluid bag 5, and the other end thereof is connected to one end of the additional fluid infusion circuit 822 through the connection tube 85b.

One end of the fluid infusion/drainage tube circuit 33 is connected to the other end of the fluid infusion/drainage circuit 823 through the connection tube 85c, and the other end thereof is connected to the dialysis catheter 7 through a transfer tube set 36. One end of the fluid drainage tube circuit 34 is connected to the other end of the fluid drainage circuit 824 through the connection tube 85d, and the other end thereof is connected to the drained fluid tank 6.

When the cassette 8 is mounted in the dialysis apparatus body 2, the fluid infusion tube circuit 31, the additional fluid infusion tube circuit 32, the fluid infusion/drainage tube circuit 33, and the fluid drainage tube circuit 34, each of which is connected to the switching cassette circuit 82 as the flow passage switching portion, are located on the front surface or on a front half of the side surface of the dialysis apparatus body 2.

The branch tube circuits 35, the additional fluid infusion tube circuit 32, the fluid infusion/drainage tube circuit 33, and the fluid drainage tube circuit 34 each have a klemme (forceps) 37 as flow passage opening/closing means for opening/closing the corresponding flow passage.

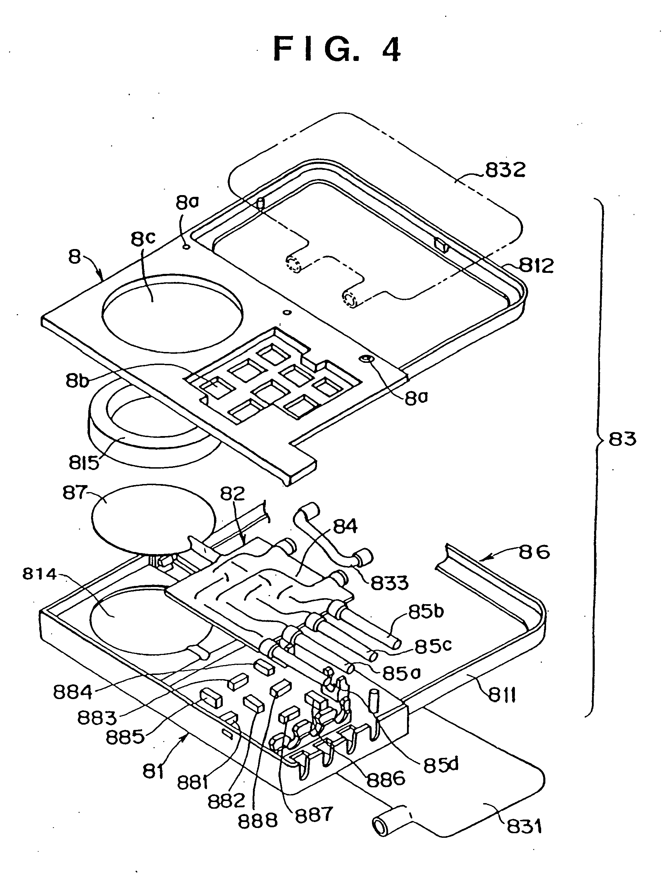

FIG. 4 is a three-dimensional exploded view of the cassette 8. In this figure, the components described above are denoted by the same reference numerals, and the detailed description thereof is omitted. The gap 86 is formed between two divisional heating cassette circuits 831 and 832. When the cassette body 81 is mounted in the cassette mounting portion 21 of the dialysis apparatus body 2, heaters (heating portions) of heating means 9 are located on both sides (upper and lower sides) of each of the divisional heating cassette circuits 831 and 832, so that each of the divisional heating cassette circuits 831 and 832 is heated in a state being sandwiched between the corresponding heaters.

The cassette body 81 has the switching cassette circuit 82 shown in FIG. 1. The switching cassette circuit 82 is composed of the fluid infusion circuit 821, the additional fluid infusion circuit 822, the fluid infusion/drainage circuit 823, and the fluid drainage circuit 824 shown in FIG. 3. The other end of the additional fluid infusion circuit 822 communicates with a middle portion of the fluid infusion circuit 821, and one end of the fluid drainage circuit 824 communicates with a portion near the other end of the fluid infusion circuit 821.

When the cassette body 81 is mounted in the cassette mounting portion 21 of the dialysis apparatus body 2, the switching cassette circuit 82 is switchable between a fluid infusion circuit state and a fluid drainage circuit state by a closing operation using the clampers 240 shown in FIG. 3.

The term "fluid infusion circuit state" used herein means a state that the fluid infusion circuit 821 (or additional fluid infusion circuit 822) communicates with the fluid infusion/drainage circuit 823, to thereby communicate the dialysis fluid bags 4 (or additional dialysis fluid bag 5) to the dialysis catheter 7, and therefore, means a state necessary for infusing the dialysis fluid to the inside of the peritoneum of the patient K, that is, a state allowing infusion of the dialysis.

The term "fluid drainage circuit state" used herein means a state that the fluid infusion/drainage circuit 823 communicates to the fluid drainage circuit 824, to thereby communicate the dialysis catheter 7 to the drained fluid tank 6, and therefore, means a state necessary for draining the dialysis fluid from the inside of the peritoneum of the patient K, that is, a state allowing drainage of the dialysis fluid. The cassette body 81 also has the heating cassette circuit 83 shown in FIG. 4. The heating cassette circuit 83 has the two sheet-like divisional heating cassette circuits 831 and 832 provided opposite to each other.

One end of the lower divisional heating cassette circuit 831 communicates with the other end of the fluid infusion circuit 821, and the other end thereof communicates with one end of the upper divisional heating cassette circuit 832 through a connection pipe 833. The other end of the upper divisional heating cassette circuit 832 communicates with one end of the fluid infusion/drainage circuit 823.

The dialysis fluid thus sequentially flows through the lower and upper divisional heating cassette circuits 831 and 832 in this order.

In preferred embodiments, the flow of the dialysis fluid may be divided into two parts running through the lower and upper divisional heating cassette circuits 831 and 832, and thereafter, the divided parts of the flow may be combined with each other.

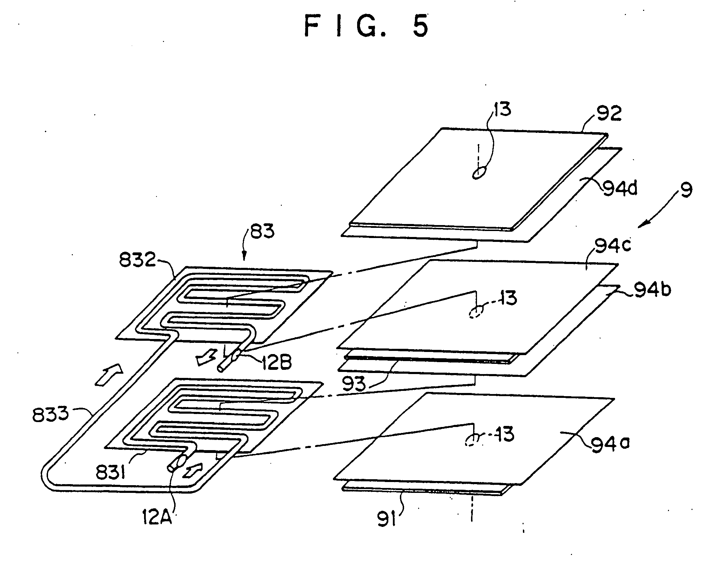

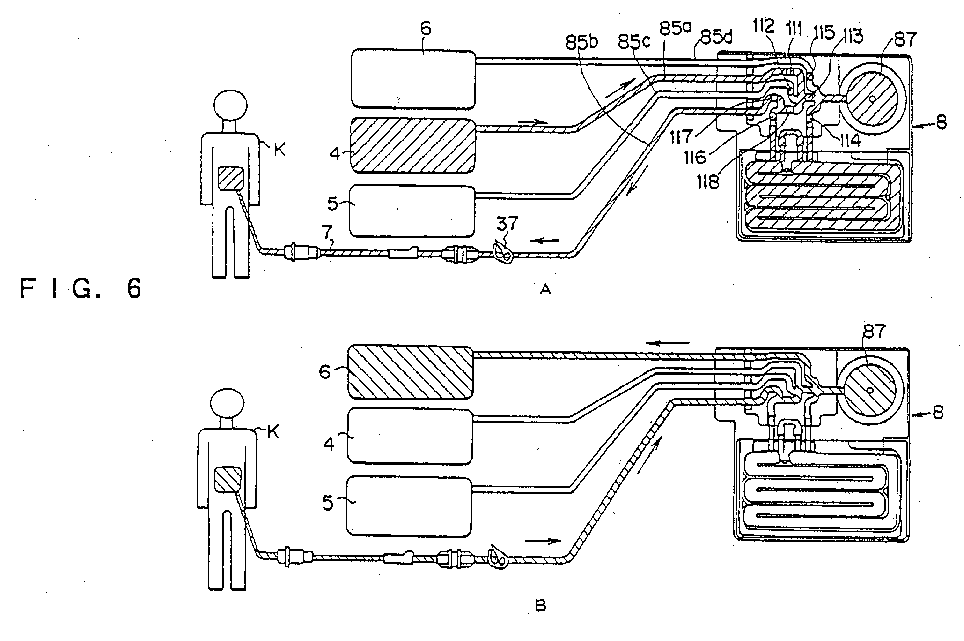

The flow passages of the divisional heating cassette circuits 831 and 832 meander as shown in the plan view of the cassette 8 of FIG. 5 and the rear view of the cassette 8 of FIG. 6. Alternatively, the flow passages of the divisional heating cassette circuits 831 and 832 may swirl. By arranging the flow passages of the divisional heating cassette circuits 831 and 832 into such meandering or swirling shapes, the flow passages of the divisional heating cassette circuits 831 and 832 become long, thereby allowing reliable heating of the dialysis fluid.

The cassette body 81 is provided with a diaphragm pump 87 which is held in an airtight state in a pump chamber (to be described later) in order to perform pumping action by its contraction and expansion to deliver the dialysis fluid. The diaphragm pump 87 is connected to a middle portion of the fluid infusion circuit 821.

The diaphragm pump 87 is contained in an airtight manner in the pump chamber by a flange member 815. When a pressure in the pump chamber is increased, the diaphragm pump 87 is contracted, whereas when the pressure in the pump chamber is reduced, the diaphragm pump 87 is expanded.

The cassette body 81 has, as described above, the bypass circuit 84. One end of the bypass circuit 84 is connected to the upstream side of the heating cassette circuit 83, preferably, to a middle portion of the fluid infusion circuit 821 as in this embodiment, and the other end thereof is connected to the downstream side of the heating cassette circuit 83, preferably, to a middle portion of the fluid infusion/drainage circuit 823 as in this embodiment. The bypass circuit 84 connects the upstream and downstream sides of the heating cassette circuit 83 to each other, thus forming a cycling circuit for cooling the dialysis fluid.

The bypass circuit 84 may have a forcedly cooling means such as a Peltier element for forcedly cooling the dialysis fluid. The dialysis fluid flowing in the bypass circuit 84 can be thus cooled quickly and reliably.

The switching cassette circuit 82, the heating cassette circuit 83, the bypass circuit 84, and the diaphragm pump 87 are arranged substantially within a plane. This is advantageous in further reducing the thickness of the cassette 8.

When the cassette body 81 is mounted in the cassette mounting portion 21 of the dialysis apparatus body 2, the outlet side (downstream side) of the heating cassette circuit 83 is switchable between a final fluid infusion circuit state and a return circuit state. The term "final fluid infusion circuit state" used herein means a state that the outlet side of the heating cassette circuit 83 communicates with the fluid infusion/drainage circuit 823 and does not communicate with the bypass circuit 84. The term "return circuit state" used herein means a state that the outlet side of the heating cassette circuit 83 communicates with the bypass circuit 84 and does not communicate with the fluid infusion/drainage circuit 823.

As shown in the three-dimensional exploded view of the cassette in FIG. 4, first to eighth support projections 881 to 888 for forming flow passage switching portions are formed at positions, corresponding to the switching cassette circuit 82, of the lower body frame 811. The first support projection 881 supports a portion near one end of the fluid infusion circuit 821. The second support projection 882 supports the additional fluid infusion circuit 822. The third support projection 883 supports a portion, located between the diaphragm pump 87 and one end of the bypass circuit 84, of the fluid infusion circuit 821. The fourth support projection 884 supports a portion, located between the diaphragm pump 87 and one end of the heating cassette circuit 83, of the fluid infusion circuit 821. The fifth support projection 885 supports the fluid drainage circuit 824. The sixth support projection 886 supports a portion, located between the other end of the heating cassette circuit 83 and the other end of the bypass circuit 84, of the fluid infusion/drainage circuit 823. The seventh support projection 887 supports a portion near the other end of the fluid infusion/drainage circuit 823. The eighth support projection 888 supports the bypass circuit 84.

The switching cassette circuit 82, the bypass circuit 84, and the diaphragm pump 87 are integrally formed by blow molding. This makes it possible to eliminate the need of preparing separate parts and joining the separate parts to each other. This improves the quality of the cassette 8 and also reduces the production cost thereof.

The divisional heating cassette circuits 831 and 832 of the heating cassette circuit 83 are formed by sheet molding. This simplifies the production of the divisional heating cassette circuits 831 and 832 and reduces the cost thereof.

The switching cassette circuit 82, the bypass circuit 84, and the diaphragm pump 87 are joined to the divisional heating cassette circuits 831 and 832 by RF fusion (RF welding) or adhesive bonding.

One example of forming each of the divisional heating cassette circuits 831 and 832 by sheet molding involves stacking two resin sheets to each other and partially fusing the resin sheets to each other in a specific flow passage pattern. In this case, portions remaining as not fused form a flow passage in each of the divisional heating cassette circuits 831 and 832.

Each of the switching cassette circuit 82, the heating cassette circuit 83, the bypass circuit 84, and the diaphragm pump 87 is preferably made from a soft resin. Examples of the soft resins include polyolefins such as polyethylene, polypropylene, an ethylenepropylene copolymer, and an ethylene-vinyl acetate copolymer (EVA); polyesters such as polyvinyl chloride, polyvinylidene chloride, polystyrene, polyamide, polyimide, poly-(4-methylpentene-1), ionomer, acrylic resin, polyethylene terephthalate (PET), and polybutylene terephthalate (PBT); various thermoplastic elastomers such as a styrene-based elastomer, a polyolefin-based elastomer, a polyvinyl chloride-based elastomer, a polyurethane-based elastomer, a polyester-based elastomer, and a polyamide-based elastomer; silicone resin; polyurethane; and copolymers, blends, and polymer alloys, which contain the above resins as main components. These materials may be used not only singly but also in combination. For example, a stacked body having two layers made from different materials selected from the above resins may be used.

The cassette body 81 has positioning holes 8a for positioning the cassette body 81 to the sub-base 201 by inserting positioning pins of the sub-base 201 in the positioning holes 8a enable positioning with positioning pins (to be described later). The cassette body 81 also has openings 8b at positions corresponding to those of the first to eighth projections 881 to 888. The openings 8b serves as part of the flow passage switching portion. The clampers 240 pass through the openings 8b, to close the flow passages.

As shown in the heater arrangement view of FIG. 5, the heating means 9 for heating the heating cassette circuit 83 of the cassette 8 is formed in the dialysis apparatus body 2. The heating means 9 has a plate-like (layer-like) lower sheet heater 91, a plate-like (layer-like) upper sheet heater 92, and a plate-like (layer-like) intermediate sheet heater 93.

The lower sheet heater 91 heats the back surface of the lower divisional heating cassette circuit 831 from below through an aluminum plate 94a serving as a heat transfer member. The upper sheet heater 92 heats the top surface of the upper divisional heating cassette circuit 832 from above through an aluminum plate 94d serving as a heat transfer member. The intermediate sheet heater 93 is located in the gap 86, and heats the top surface of the lower divisional heating cassette circuit 831 from above through an aluminum plate 94b serving as a heat transfer member and also heats the back surface of the upper divisional heating cassette circuit 832 from below through an aluminum plate 94c serving as a heat transfer member.

Accordingly, the dialysis fluid in the lower divisional heating cassette circuit 831 is heated in a state being sandwiched between the lower and intermediate sheet heaters 91 and 93, and the dialysis fluid in the upper divisional heating cassette circuit 832 is heated in a state being sandwiched between the upper and intermediate sheet heaters 92 and 93. This improves the heating efficiency of the dialysis fluid in the heating cassette circuit 83 by the heating means 9, which leads to an advantage in reducing the size and weight of the dialysis apparatus body 2 and cassette 8.

Clamp means 11 shown in FIG. 3 switches the state of the switching cassette circuit 82 of the cassette 8 from one of the fluid infusion circuit state and fluid drainage circuit state to the other, and also switches the state of the outlet side of the heating cassette circuit 83 from one of the final fluid infusion circuit state and fluid drainage circuit state to the other. The clamp means 11 further aids pumping of the diaphragm pump 87.

To be more specific, first to eighth clamps 111 to 118 indicated by arrows are formed in the dialysis apparatus body 2. The first clamp 111 cooperates with the first support projection 881 to clamp a portion near one end of the fluid infusion circuit 821 so as to close the flow passage. The second clamp 112 cooperates with the second support projection 882 to clamp the additional fluid infusion circuit 822 so as to close the flow passage. The third clamp 113 cooperates with the third support projection 883 to clamp a portion, located between the diaphragm pump 87 and one end of the bypass circuit 84, of the fluid infusion circuit 821 so as to close the flow passage. The fourth clamp (pumping control clamp) 114 cooperates with the fourth support projection 884 to clamp a portion, located between the diaphragm pump 87 and one end of the heating cassette circuit 83, of the fluid infusion circuit 821 so as to close the flow passage.

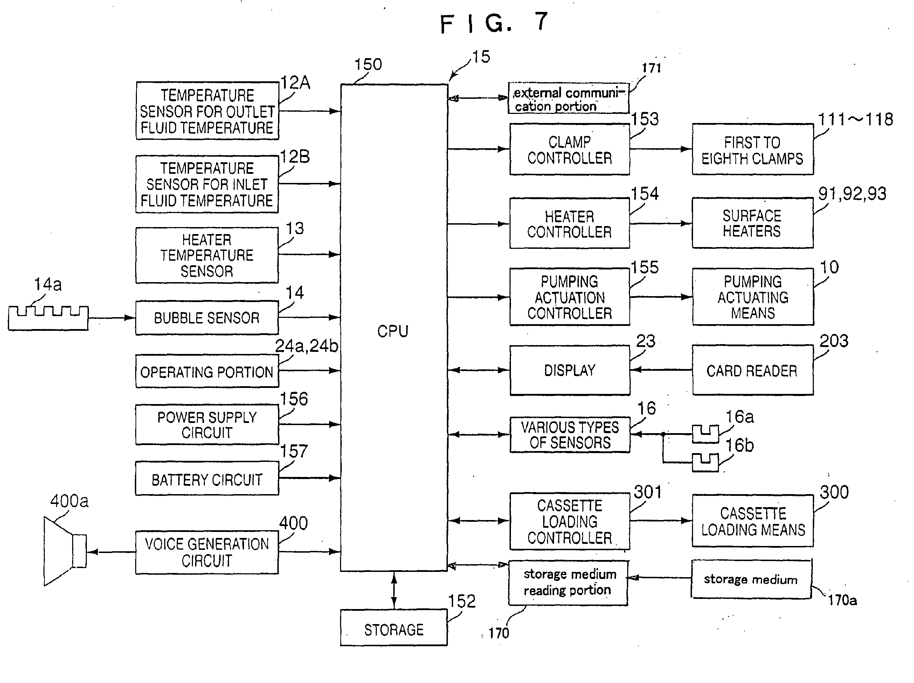

Similarly, the fifth clamp 115 cooperates with the fifth support projection 885 to clamp the fluid drainage circuit 824 so as to close the flow passage. The sixth clamp 116 cooperates with the sixth support projection 886 to clamp a portion, located between the other end of the heating cassette circuit 83 and the other end of the bypass circuit 84, of the fluid infusion/drainage circuit 823 so as to close the flow passage. The seventh clamp 117 cooperates with the seventh support projection 887 to clamp a portion near the other end of the fluid infusion/drainage circuit 823, so as to close the flow passage. The eighth clamp 118 cooperates with the eighth support projection 888 to clamp the bypass circuit 84 so as to close the flow passage. In the case of switching the switching cassette circuit 82 to the fluid infusion circuit state, the first clamp 111 (or second clamp 112), the fourth clamp (pumping control clamp) 114, the sixth clamp 116, and the seventh clamp 117 are switched to the unclamp states, and the second clamp 112 (or first clamp 111), the fifth clamp 115, and the eighth clamp 118 are switched to the clamp states. In the case of pressurizing the inside of a chamber 814 by pumping actuating means 10 (see FIG. 7), the fourth clamp 114 is switched to the unclamp state, and the third clamp 113 is switched to the clamp state. In the case of reducing the pressure in the chamber 814 with the pumping actuating means 10, the fourth clamp 114 is switched to the clamp state, and the third clamp 113 is switched to the unclamp state. As a result, the dialysis fluid can be delivered, that is, infused from the dialysis fluid bags 4 (or additional dialysis fluid bag 5) toward the dialysis catheter 7, thus obtaining a state allowing the delivery of the dialysis fluid in the peritoneal cavity as shown in FIG. 6A.

In the case of switching the switching cassette circuit 82 to the fluid drainage circuit state, the seventh and eighth clamps 117 and 118 are switched to the unclamp states, and the first, second, fourth, and sixth clamps 111, 112, 114, and 116 are switched to the clamp states, to obtain a state allowing recovery of the drained fluid as shown in FIG. 6B.

In the case of reducing the pressure in the pump chamber 814 with the pumping actuating means 10, the third clamp 113 is switched to the unclamped state, and the fifth clamp 115 is switched to the clamp state. In the case of pressurizing the inside of the chamber 814 by the pumping actuating means 10, the third clamp 113 is switched to the clamp state, and the fifth clamp 115 is switched to the unclamp state, thereby allowing the dialysis fluid to be drained from the dialysis catheter 7 toward the drained fluid tank 6.

The diaphragm pump 87, the third to fifth clamps 113 to 115, and the pumping actuating means 10 constitute fluid delivery (infusion) means for delivering the dialysis fluid.

When the switching cassette circuit 82 is in the fluid infusion circuit state and the outlet side of the heating cassette circuit 83 is in the final fluid infusion circuit state, the seventh clamp 117 is in the unclamp state, while the eighth clamp 118 is in the clamp state.

In the case of switching the outlet side of the heating cassette circuit 83 to the return circuit state, the first, second, and seventh clamps 111, 112, and 117 are switched to the clamp states, and the eighth clamp 118 is switched to the unclamp state. As a result, the dialysis fluid does not flow from the outlet side of the heating cassette circuit 83 toward the dialysis catheter 7, but flows through the bypass circuit 84 toward the diaphragm pump 87. In other words, the dialysis fluid circulates between the bypass circuit 84 and heating cassette circuit 83.

The seventh and eighth clamps 117 and 118 constitute fluid infusion/drainage circuit switching means for switching the state of the outlet side of the heating cassette circuit 83 to one of the final fluid infusion circuit state and the return circuit state to the other.

In the case of draining the dialysis fluid, the drained fluid is recovered in the drained fluid tank 6 through the bypass circuit 84. This simplifies the configuration of the flow passage.

By providing the switching cassette circuit 82, the heating cassette circuit 83, the bypass circuit 84, and the diaphragm pump 87 on the cassette body 81 as described above, the size and weight of the peritoneal dialysis apparatus 1 can be reduced. As a result, it is possible to facilitate handling such as transportation of the peritoneal dialysis apparatus 1, and hence to realize smooth medical care.

In particular, since the dialysis fluid flowing through the divisional heating cassette circuits 831 and 832 is heated in the state that each of the divisional heating cassette circuits 831 and 832 is sandwiched by the corresponding heaters, the heating efficiency of the dialysis fluid can be improved. As a result, it is possible to further reduce the size and weight of the peritoneal dialysis apparatus 1.

On the other hand, as shown in FIG. 2, the peritoneal dialysis apparatus 1 has various types of sensors typically used for temperature management of the dialysis fluid.

To be more specific, in the dialysis apparatus body 2, a temperature sensor 12A for measuring (detecting) the temperature (outlet fluid temperature) of the dialysis fluid flowing through the outlet side (downstream side) of the heating cassette circuit 83 is provided on the downstream side of the heating cassette circuit 83, and a temperature sensor 12B for measuring (detecting) the temperature (inlet fluid temperature) of the dialysis fluid flowing through the inlet side (upstream side) of the heating cassette circuit 83 is provided on the upstream side of the heating cassette circuit 83.

A preferred example of each of the temperature sensors 12A and 12B is a thermopile infrared sensor (noncontact type temperature sensor) with a very quick response speed. With such sensors, the temperatures of the sheet heaters 91, 92, and 93 can be controlled at high precision.

As shown in FIG. 5, three heater temperature sensors 13 represented by thermisters for measuring (detecting) the temperatures of the sheet heaters 91, 92, and 93 are mounted on the sheet heaters 91, 92, and 93, respectively. Two bubble sensors 14 for detecting bubbles on the inlet and outlet sides of the switching cassette circuit 82 are mounted to the dialysis apparatus body 2. It is to be noted that only one of the bubble sensors, denoted by the reference numeral 14a, is shown in FIG. 1. The peritoneal dialysis apparatus 1 has a closure sensor for detecting closure of a circuit, and various types of other sensors, for example, the sensors 16a and 16b shown in FIG. 1.

As shown in a block diagram of FIG. 7, the peritoneal dialysis apparatus 1 has a control system (control means) 15 for controlling infusion, drainage, and the like of the dialysis fluid.

The control system 15 has a CPU (Central Processing Unit) 150 and a storing unit 152. The CPU 150 is electrically connected to a clamp controller 153 for controlling the plurality of clamps 111 to 118, a heater controller 154 for controlling the temperatures of the plurality of sheet heaters 91, 92, and 93, and a pumping actuation controller 155 for controlling the pumping actuating means 10. The CPU 150 is also electrically connected to the temperature sensor 12A for measuring the outlet fluid temperature, the temperature sensor 12B for measuring the inlet fluid temperature, the heater temperature sensors 13, the bubble sensors 14, the display portion 23, and the operating portions 24a and 24b. The CPU 151 is also electrically connected to a power supply circuit 156, a battery circuit 157, a voice generation circuit 400, and a cassette loading controller 301 for controlling cassette loading means 300. The display portion 23 is electrically connected to the card reader 203 in which the above-described memory card is loadable. The CPU 150 is also electrically connected to a storage medium reading portion 170 for reading out data from a storage medium 170a in which various kinds of control programs have been stored, such as a flexible disk and a CD-ROM. The CPU 150 also has an external communication portion 171 which allows intercommunication with a portable terminal 1000 having a screen 1023 (see FIG. 1), a medical cite, or the like by way of LAN, Internet, radio communication such as infrared communication.

When the temperature measured by the temperature sensor 12A for measuring the outlet fluid temperature becomes equal to or more than a predetermined value (39°C in this embodiment), the control system 15 drives the clamp controller 153 and the heater controller 154 such that the seventh clamp 117 is switched to the clamp state and the eighth clamp 118 is switched to the unclamped state by the clamp controller 153, and that the plurality of sheet heaters 91, 92, and 93 are switched to the OFF states, that is, the drive of the plurality of sheet heaters 91, 92, and 93 is stopped by the heater controller 154.

Outputs (output values) from the sheet heaters 91, 92, and 93 are selected on the basis of the temperature control flow of the dialysis fluid and the temperature of the dialysis fluid. More specifically, on the basis of the temperatures measured by the outlet liquid temperature sensor 12A and the inlet liquid temperature sensor 12B, the control system 15 controls the outputs (driving operations) of the plurality of sheet heaters 91, 92, and 93 such that the temperature of the dialysis fluid to be infused becomes a value within a specific temperature range. The clamp controller 153 switches the first clamp 111 (or second clamp 112) and the fourth, sixth, and seventh clamps 114, 116, and 117 to the unclamp states, and switches the second clamp 112 (or first clamp 111) and the fifth and eighth clamps 115 and 118 to the clamp states. The switching cassette circuit 82 can be thus switched to the fluid infusion circuit state. The heater controller 154 performs control operation to supply power (output) to the plurality of sheet heaters 91, 92, and 93. The heating step of heating the dialysis fluid flowing through the heating cassette circuit 83 is thus prepared. In other words, the dialysis fluid temperature control flow enters the preheat step.

When a time T1 has elapsed since the supply of a power to the plurality of sheet heaters 91, 92, and 93 was started, the preheat step is ended. After the preheat step is ended, the pumping actuation controller 155 controls the pumping actuating means 10 so as to alternately repeat the switching between pressurization and depressurization of the inside of the pump chamber 814. At the same time, the clamp controller 153 controls the fourth clamp 114 so as to alternately repeat the switching of the fourth clamp 114 between the clamp state and unclamp state in accordance with the switching between pressurization and depressurization of the inside of the chamber 814, and also controls the third clamp 113 to alternately repeat the switching of the third clamp 113 between the clamp state and unclamp state in accordance with the switching between pressurization and depressurization of the inside of the chamber 814. This causes the pumping action (deflation and inflation) of the diaphragm pump 87, to deliver (infuse) the dialysis fluid from the dialysis fluid bags 4 toward the dialysis catheter 7.

After the preheat step is ended, the dialysis fluid temperature control flow goes on to an initial heating step, and then goes on to a normal heating step. In the normal heating step, if the temperature measured by the outlet fluid temperature sensor 12A is less than 33°C, the output control for the plurality of sheet heaters 91, 92 and 93 is performed such that a heater output value obtained by P control (proportional control) is outputted to the plurality of sheet heaters 91, 92, and 93.

If the temperature measured by the outlet fluid temperature sensor 12A is 33°C or more and less than 39°C, a heater output value obtained by PI control (proportional-integral control) is outputted to the plurality of sheet heaters 91, 92, and 93.

The output control of the plurality of sheet heaters 91, 92, and 93 can be thus highly accurately performed. In the initial heating step or normal heating step, if the temperature measured by the outlet fluid temperature sensor 12A becomes 39°C or more, the clamp controller 153 controls the seventh clamp 117 so as to switch the seventh clamp 117 to the clamp state and also controls the eighth clamp 118 so as to switch the eighth clamp 118 to unclamped state. At the same time, the heater controller 154 stops the supply of the power to the plurality of sheet heaters 91, 92, and 93, that is, switches the plurality of sheet heaters 91, 92, and 93 to the OFF states. Accordingly, the outlet side of the heating cassette circuit 83 is switched to the return circuit state. As a result, the dialysis fluid flows from the heating cassette circuit 83 not toward the dialysis catheter 7 but toward the bypass circuit 84, returns to the upstream side of the heating cassette circuit 83 through the bypass circuit 84, and circulates between the bypass circuit 84 and heating cassette circuit 83. During this circulation, the temperature of the dialysis fluid is lowered (that is, cooled). Namely, the dialysis fluid heating control flow goes on to the cooling step. With this temperature control, it is possible to prevent a dialysis fluid heated at a temperature higher than the body temperature of the patient K, more specifically, a dialysis fluid heated at 39°C or more from being infused in the patient K, and hence to perform safe dialysis therapy.

When the temperature measured by the outlet fluid temperature sensor 12A becomes less than 39°C, the clamp controller 153 controls the seventh clamp 117 to switch the seventh clamp 117 to the unclamp state and also control the eighth clamp 118 to switch the eighth clamp 118 to the clamped state. At the same time, the plurality of sheet heaters 91, 92, and 93 are switched to the ON states. Accordingly, the outlet side of the heating cassette circuit 83 is restored to the final fluid infusion circuit state, with a result that the control flow goes on to the initial heating step or normal heating step again. After a predetermined amount of dialysis fluid is infused (injected) to the inside of the peritoneum of the patient K, the step of infusing the dialysis fluid is ended.

After the step of infusing the dialysis fluid is ended, the clamp controller 153 controls the seventh and eighth clamps 117 and 118 to switch the seventh and eighth clamps 117 and 118 to the unclamp states, and also controls the fourth and sixth clamps 114 and 116 to switch the fourth and sixth clamps 114 and 116 to the clamp states. Accordingly, the switching cassette circuit 82 is switched to the fluid drainage circuit state.

The pumping actuation controller 155 controls the pumping actuating means 10 to alternately repeat the switching between depressurization and pressurization of the inside of the chamber 814. The clamp controller 153 controls the third clamp 113 to alternately repeat the switching of the third clamp 113 between the unclamp state and clamp state in accordance with the switching between depressurization and pressurization of the inside of the chamber 814, and also controls the fifth clamp 115 to alternately repeat the switching of the fifth clamp 115 between the clamp state and unclamp state in accordance with the switching between depressurization and pressurization of the inside of the chamber 814. This causes the pumping action of the diaphragm pump 87, to deliver (drain) the dialysis fluid in the peritoneum from the dialysis catheter 7 toward the drained fluid tank 6.

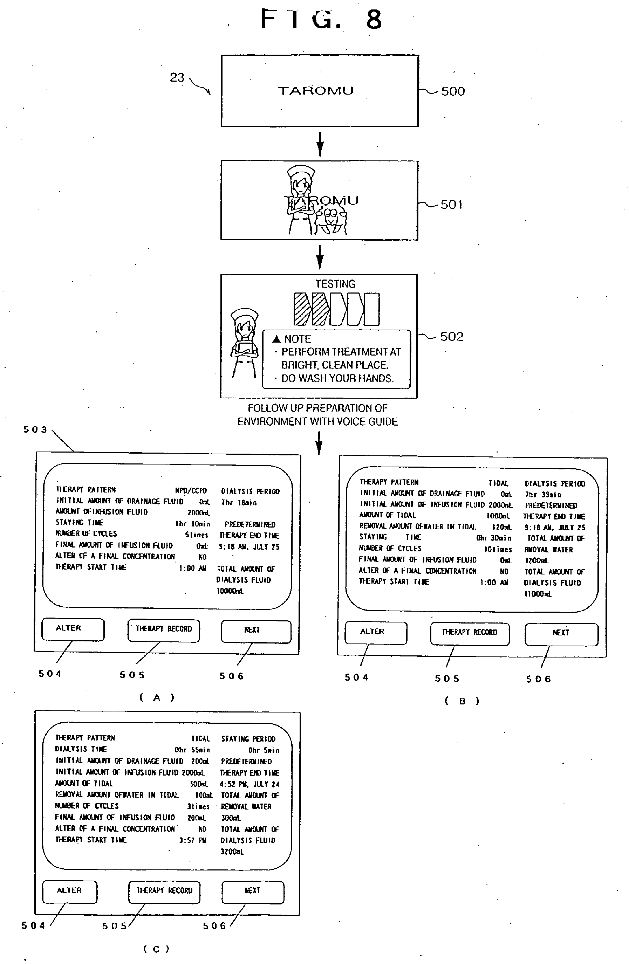

FIGS. 8 to 13 are diagrams showing sequentially variable display screens on the display portion 23 shown in FIG. 1.

In the block diagram of FIG. 8, when the power supply of the apparatus 2 is turned on and the operating portion 24a (see Fig.1) is depressed, an initial screen 500 showing the maker's name of the apparatus appears on the display portion 23. The initial screen 500 is changed to a screen 501 on which a guide nurse and a sheep (character image) are displayed in color. The screen 501 is changed to a screen 502 on which moving arrows indicating that the storage 152 is being initialized are displayed. At the same time, a voice guide "Perform therapy at bright, clean place. Wash your hands." Or the like is given in the form of a message of electronic sound generated from the loudspeaker 400a. The screen 502 is then automatically changed to a screen 503 on which the previous therapy conditions stored in the storing portion 152 are displayed.

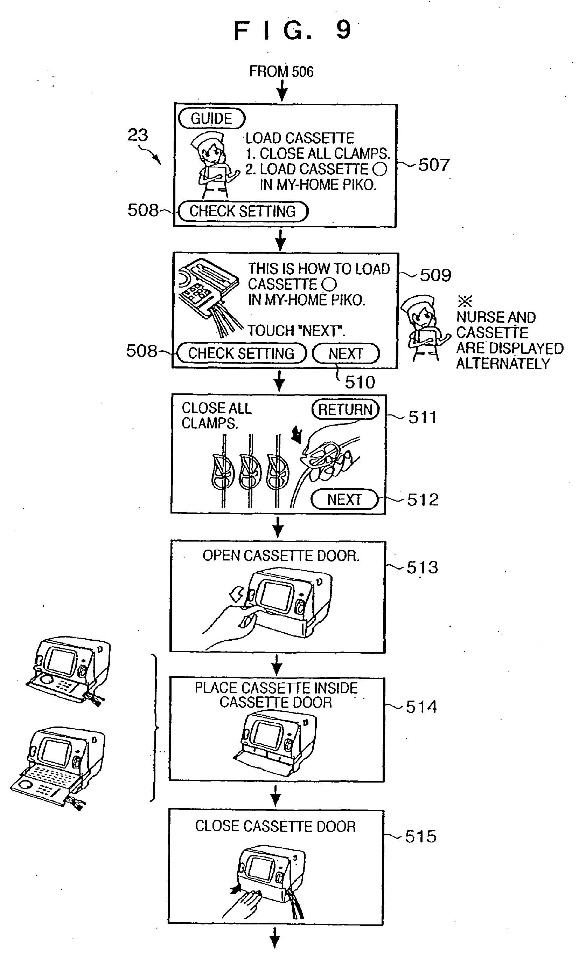

FIG. 8A shows one example that therapy conditions in a first therapy mode (or so-called A mode) adapted to calculate a total amount of a dialysis fluid on the basis of inputted set-up parameters: an amount of infusion fluid, a staying time(a staying duration), and the number of cycles. In this example, parameters necessary for peritoneal dialysis are displayed as the previous data. These parameters are represented by a therapy pattern (NPD/CCPD), an initial amount of drained fluid (0 mL), an amount of infusion fluid (2,000 mL), a staying time (1 hr and 10 min) in the peritoneal cavity, the number of cycles (5 times), a final amount of infusion fluid (0 mL), alter of a final concentration (NO), a therapy (dialysis) start time (1:00 AM), a dialysis time(a dialysis duration) (7 hr and 18 min), an estimated therapy (dialysis) end time (9:18, July 25), and a total amount of dialysis fluid (10,000 mL). If the patient is intended to perform the current therapy under these previous conditions, the patient may depress a touch key 506 "NEXT" to jump the screen 503 to a screen 507 in FIG. 9.

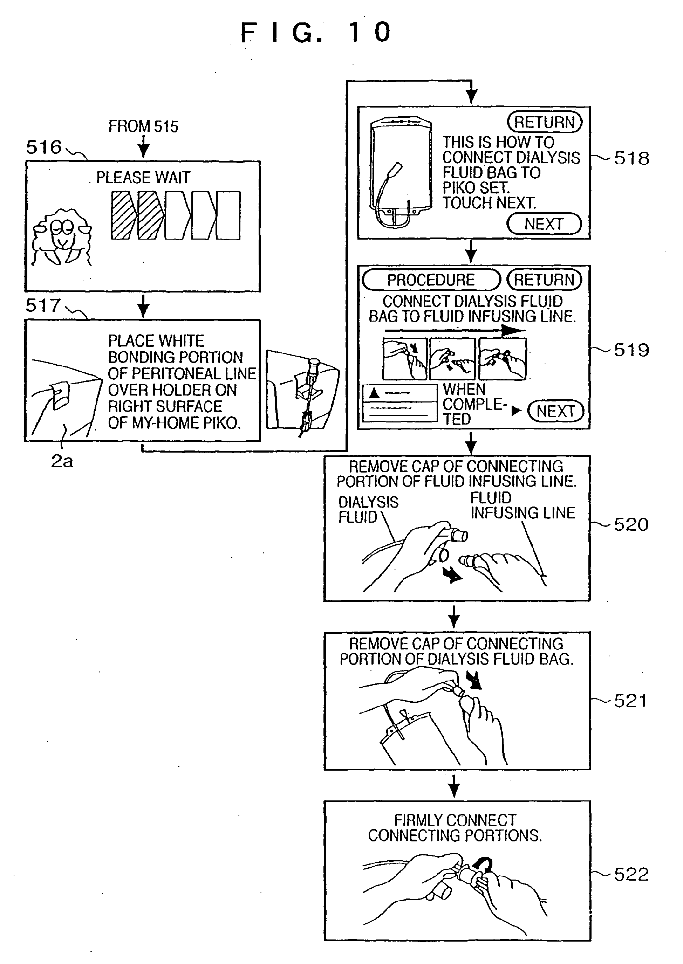

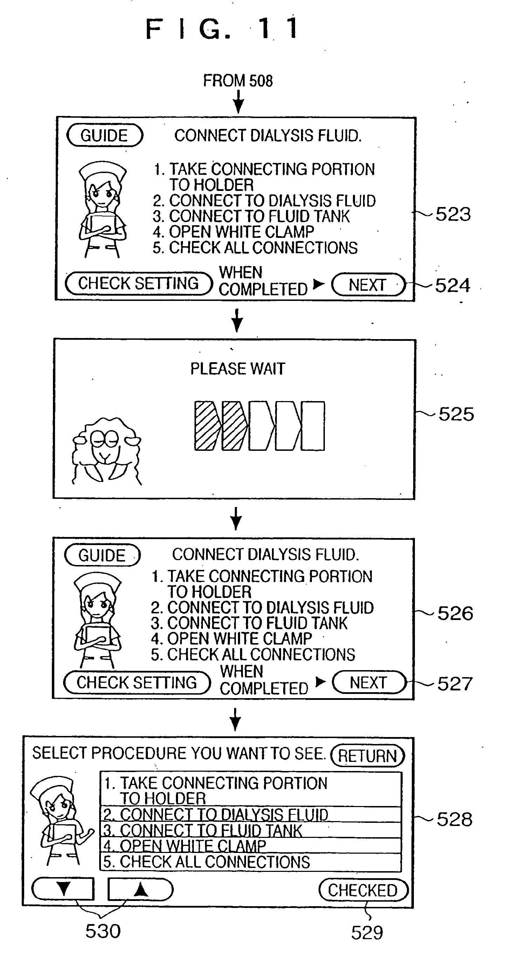

On the screen 507 shown in FIG. 9, a message "Set the cassette 8 in the mounting portion of the apparatus." is displayed together with the corresponding voice guide, and simultaneously a touch key 508 "CHECK SETTING" is displayed. If the touch key 508 is depressed, the screen 507 is jumped to a screen 523 in FIG. 11. On a screen 509 following the screen 507, a voice guide "This is a procedure how to load the cassette in the ***My-home Piko***" (Trademark of Terumo Corporation) is given, and about that time, a cartooned nurse and a cassette are displayed alternately. It is to be noted that the "***My-home Piko***" is an assembly of the cassette 8 and piping as shown in FIG. 6. If a touch key 510 "NEXT" is depressed, the screen 509 is changed to a screen 511 on which a message "Close all the clampers" and the clamper closing procedure shown by a color still image are displayed together with the corresponding voice guide. If a touch key 512 "NEXT" is depressed, the screen 511 is changed to a screen 513 on which an image indicating that the lid member 22 is opened on the patient's side is displayed together with the corresponding voice guide, and then the screen 513 is automatically changed to a screen 514. On the screen 514, a moving image (animating image) showing how to insert the cassette 8 through over the lid member of the apparatus is displayed in color. The screen 514 is changed to a screen 515 on which an image showing how to close the lid member after the cassette has been loaded is displayed together with the corresponding voice guide. The screen 515 is changed to a screen 516 shown in FIG. 10, on which a message "Please wait a minute" is displayed. This message means the stand-by state until the cassette is moved so as to be surrounded by the above-described three heater layers. The screen 516 is changed to a screen 517 on which a moving image (animating image) showing how to set the connection tunes to the hook 2a of the cassette body is displayed together with the corresponding voice guide. The screen is then changed to a screen 518.

On the screen 518, a message "Connect the dialysis fluid bag to the ***Piko*** set (Trademark of Terumo Corporation) 8" is displayed together with the corresponding voice guide. When a touch key "NEXT" is depressed, the screen 518 is changed to a screen 519 on which an image showing the connection state is displayed together with the corresponding voice guide. The screen 519 is automatically changed to screens 520, 521, and 522 in sequence, to indicate an operation necessary for connection. Screens 523, 525, 526, and 528 in FIG. 11 are those sequentially appearing when the touch key 508 for "CHECK SETTING" in the screen 507 or 508 in FIG. 9 is depressed. On the screen 523, a message for checking the connection is displayed together with the corresponding voice guide. When a touch key 524 "NEXT" is depressed after completion of the connection, the screen 523 is changed to a stand-by screen 525 and is further changed to a screen 526. On the screen 526, an image showing an unclamp state and the like is displayed together with the corresponding voice guide. When a touch key 527 "NEXT" is depressed, the screen 526 is changed to a screen 528 on which a touch key "SETTING" is depressed for preparation of dialysis. After that, dialysis is started by depressing the operating portion 24a. On the screen 528, one of items 1 to 5 displayed on the screen 528 is selectively highlighted by depressing either a touch key 530 marked with an upward arrow or another touch key 530 marked with a downward arrow, and the selected item is established by depressing a touch key "RETURN", to confirm the content of the selected item. On the screen 526, one of items, details of which are required for the patient, can be selected by depressing a touch key 530.

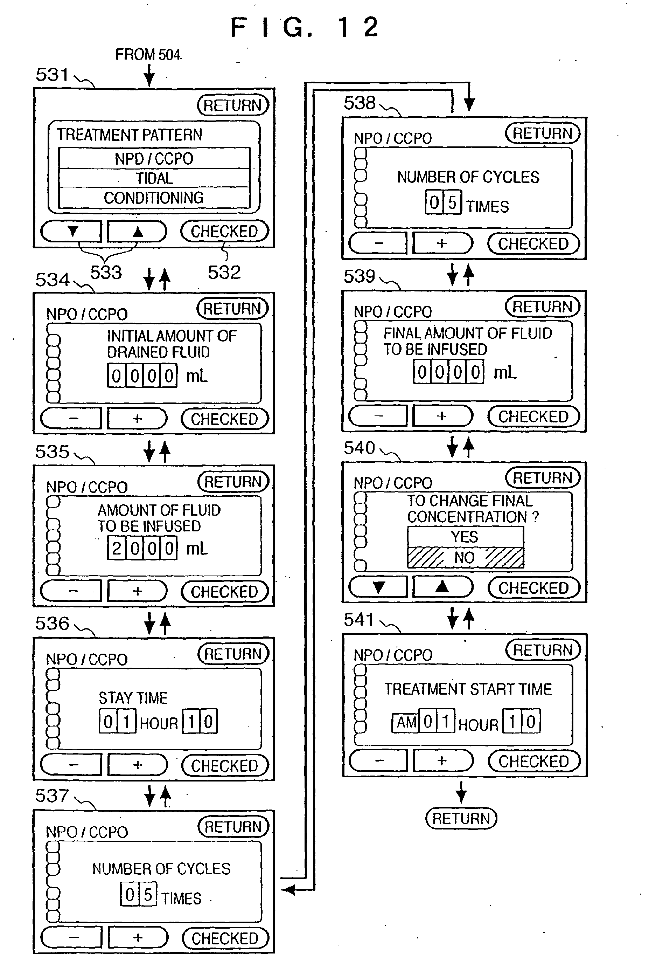

On the other hand, when a touch key 504 "ALTER" on the screen 503 in FIG. 8A is depressed, the screen 503 is changed to a screen 531 in FIG. 12. In this example, on the screen 531, "NPD/CCPD" is selected as the therapy pattern, characters "NPD/CCPD" are displayed on the screen 531. If "Tidal" is selected as the therapy pattern, characters "Tidal" are displayed on the screen 531. One of screens 534 to 541 is selected by depressing either a touch key 533 marked with an upward arrow or another touch key 533 marked with a downward arrow, and a parameter displayed on the selected screen is dialogically set up. Since each of the screens 534 to 541 is displayed in black-and-white in the unchanged background color, the patient can perceive that the setting is now being changed. On the screen 541, a dialysis (therapy) start time can be set up and inputted in either a first therapy mode (or so-called A-mode) or a second therapy mode (or so-called B-mode). The first therapy mode is adapted to calculate a total amount of dialysis fluid on the basis of inputted set-up parameters: an amount of infusion fluid, a staying time, and the number of cycles. The second therapy mode is adapted to calculate a total amount of dialysis fluid and a staying time on the basis of inputted set-up parameters: a dialysis time, an amount of infused amount, and the number of cycles. It is also possible to calculate an amount of infusion fluid, a staying time, the number of cycles, and a total amount of dialysis fluid on the basis of an inputted set-up dialysis start time used as a preferential value. In the second therapy mode, after a dialysis start time is set up, a staying time per one cycle can be automatically calculated by setting up and inputting a desired dialysis end time. Accordingly, a start time can be set up for either of the therapy patterns: NPD, CCPD, "Tidal", and "Conditioning" even in the first therapy mode or the second therapy mode.

FIG. 8B shows an example that dialysis conditions in the therapy pattern "Tidal" under the first therapy mode (or A-mode) are set up, stored, and displayed. Parameters necessary for peritoneal dialysis are displayed on the displaying portion 23. The parameters are represented by a therapy pattern (Tidal), an initial amount of drained fluid (0 mL), an initial amount of infusion fluid (2,000 mL), an amount of Tidal (1,000 mL), a removal amount of water in Tidal (120 mL), a staying time (0 hr and 30 min) in a peritoneal cavity, the number of cycles (10 times), a final amount of infusion fluid (0 mL), alter of final concentration (NO), a therapy (dialysis) start time (1:00 AM), a dialysis time (7 hr and 39 min), an estimated therapy (dialysis) end time (9:18 AM, July 25), a total amount of removal water (1,200 mL), and a total amount of dialysis fluid (11,000 mL).

FIG. 8C shows an example that dialysis conditions in the therapy pattern "Tidal" under the second therapy mode (or B-mode) are set up, stored, and displayed. Parameter necessary for peritoneal dialysis are displayed on the displaying portion 23. The parameters are represented by a therapy pattern (Tidal), a dialysis time (0 hr 55 min), an initial amount of drained fluid (200 mL), an initial amount of infusion fluid (2,000 mL), an amount of Tidal (500 mL), a removal amount of water in Tidal (100 mL), a staying time (0 hr and 5 min) in a peritoneal cavity, the number of cycles (3 times), a final amount of infusion fluid (200 mL), alter of final concentration (NO), a therapy (dialysis) start time (3:57 PM), an estimated therapy (dialysis) end time (4:52 PM, July 24), a total amount of removal water (300 mL), and a total amount of dialysis fluid (32,000 mL). In the case of selecting the second therapy mode, by inputting a desired set-up dialysis time, a staying time and a dialysis end time per one cycle can be automatically calculated.

Contents of abnormal states such as closure (occlusion) of a fluid drainage line, closure (occlusion) of a fluid infusion line, defective fluid infusion, defective fluid drainage, lack of drained fluid, closure (occlusion) of an additional fluid infusion line, drop in battery voltage, drop in outside temperature drop, closure of a peritoneal line, detection of bubbles, and power failure or the like, and the corresponding operation procedures are previously stored in the storing unit 152. If the bubble sensors 14 and various sensors 16 detect an abnormality, the screen is automatically changed to a screen displaying the abnormal state, and the kind of abnormality is sequentially displayed. As a result, the user (patient) can sequentially, dialogically check a countermeasure against the abnormality.

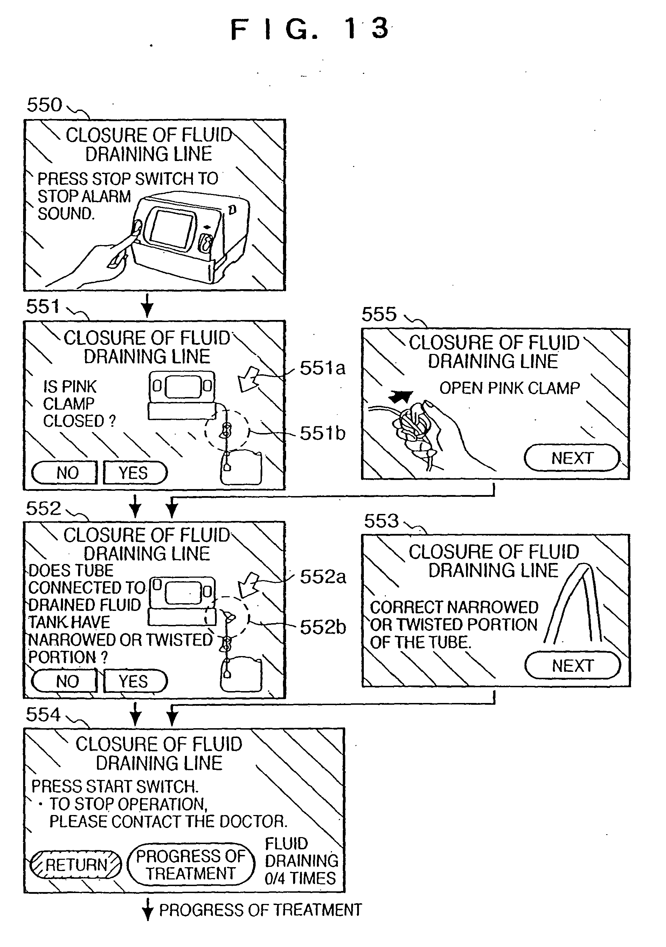

FIG. 13 shows the closure(occlusion) of the fluid drainage line as one example of abnormal states in dialysis. In order to inform the patient of the abnormal state, messages are displayed on screens 550 to 554 as still images or moving images (animating images) including characters together with corresponding voice guides. At this time, the background is displayed in a predetermined highly visible color such as yellow or orange (indicated by hatching in the figures) so as to inform the patient of the abnormal state.

On the screen 550, characters "Depress the stop-switch to stop alarm sound." are displayed on an instruction alarm screen, and simultaneously an instruction message "Depress the stop-switch to stop alarm sound." is given as a voice guide.

When the operating portion (stop-button) 24b is depressed on the basis of such an instruction, the screen 550 is changed a dialogical screen 551. One of closed locations (patterns) previously stored in the storing unit 152 is displayed by an arrow 551a and a circular dot mark 551b of a predetermined color (red) together with a schematic view of the peritoneal dialysis line, and a question "Is the pink clamp closed ?" is displayed together with the corresponding voice guide.

If "NO" is clicked on the screen 551, the screen 551 is changed to a screen 552. If "YES" is clicked on the screen 551, the screen 551 is changed to a screen 555 on which an instruction message "Open pink clamp" is given as a countermeasure together with the corresponding voice guide. If a necessary procedure is executed on the basis of such an instruction and then "NEXT" is clicked, the screen 555 is changed to the screen 552.

If "NO" is clicked on the screen 552, the screen 552 is changed to a screen 554. If "YES" is clicked on the screen 552, the screen 552 is changed to the screen 553 on which an instruction message "Correct a folded or twisted portion of the tube." as a countermeasure together with the corresponding voice guide. If a necessary procedure is executed is executed on the basis of such an instruction and then "NEXT" is clicked, the screen 553 is changed to the screen 554.

On the screen 554, a character message "Depress the start switch. To stop the operation, please contact the doctor." is displayed together with the corresponding voice guide.

In this way, since the contents and locations of abnormalities are previously estimated and stored in the storing unit 152, the content and location of an abnormality can be checked on the basis of sequential comparison with the content and location of one of the previously stored abnormalities.

On each screen, the operational state can be returned to the initial operational state by depressing the operating portion 24a.

The abnormal states include, in addition to closure of the fluid draining line shown in FIG. 13, closure of a fluid infusion line, defective fluid infusion, defective fluid drainage, lack of drained fluid, and entrapment of bubbles during therapy and priming, closure of an additional fluid infusion line during therapy and priming, battery backup due to power failure, and a drop in outer temperature. If such an abnormality occurs, the background color is changed to a highly visible color such as yellow or orange, and simultaneously a voice guide for the abnormality is given. Accordingly, even if a trouble may occur, a countermeasure against such a trouble can be easily given. The patient, therefore, can use the apparatus with confidence.

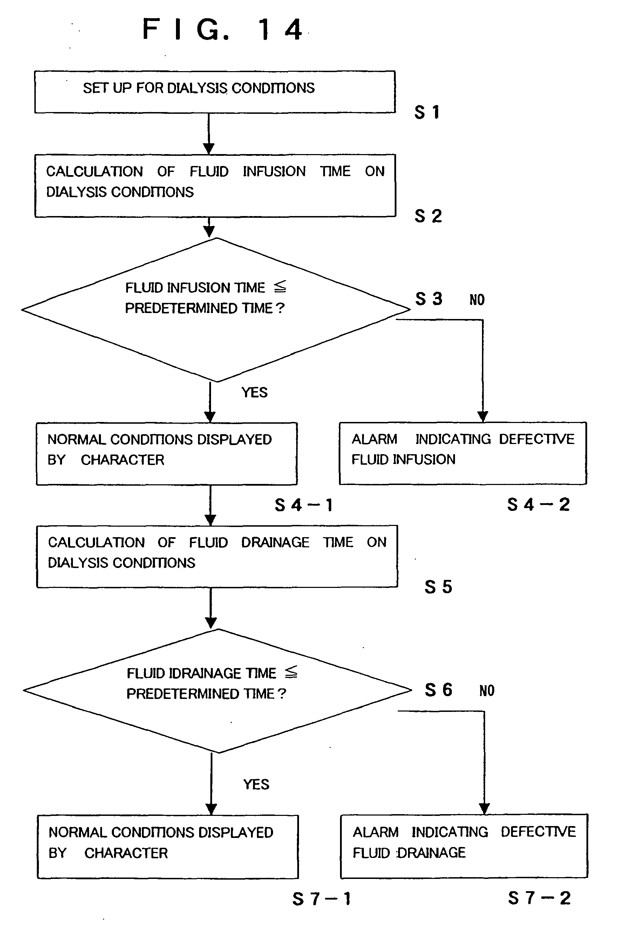

The detection of closure (occlusion) of a fluid infusion line and defective fluid infusion and an alarm for such an abnormality will be described on the basis of a flow chart shown in FIG. 14. In step S1, a therapy pattern, a staying time, the number of cycles, a dialysis time, an amount of infused amount are set-up and inputted on the set-up screens 531 to 541. In step S2, a fluid infusion time (min) is calculated on the basis of dialysis conditions such as the set-up parameter inputted in step S1 and a fluid infusion rate (mL/min) predetermined by a maker or the like. It is to be noted that the apparatus is normally configured that the user cannot set up the fluid infusion rate on the set-up screens but may be configured that the user can set up the fluid infusion rate on the set-up screens. If it is decided in step S3 that fluid infusion is performed for a fluid infusion time over a predetermined range based on the calculated fluid infusion time (min), for example, for a fluid infusion time being twice or more the calculated fluid infusion time (min), the process goes on to step S4-2. In step S4-2, an alarm indicating defective fluid infusion is displayed on the displaying portion 23 together with the corresponding voice guide. The fluid infusion time (min) is determined by adding a preparation time (min) until start of fluid infusion to a value of fluid infusion amount (mL)/fluid infusion rate (mL/min). The preparation time (min) is set, for example, to about 2 min. If the amount of infusion amount is returned to a predetermined amount of infusion amount (mL) or fluid infusion is performed at a predetermined fluid infusion rate, the process goes on to step S4-1 in which a normal image indicated by a character image is displayed on the displaying portion 23.

The detection of closure(occlusion) of a fluid drainage line and defective fluid drainage and an alarm for such an abnormality will be described on the basis of a flow chart shown in FIG. 14. First, as described above, a therapy pattern, a staying time, the number of cycles, a dialysis time, and an amount of infused amount (mL) are set-up and inputted on the set-up screens 531 to 541. An amount of drained fluid (mL) is calculated on the basis of dialysis conditions such as the above set-up parameter and a fluid infusion rate (mL/min) predetermined by a maker or the like. It is to be noted that the apparatus is generally configured that the user cannot set up the fluid infusion rate on the set-up screens but may be configured that the user can set up the fluid infusion rate on the set-up screens. In step S5, a fluid drainage time (min) is calculated on the basis of a fluid drainage rate (mL/min) predetermined by a maker or the like and the calculated amount of drained fluid (mL). It is to be noted that the apparatus is generally configured that the user cannot set up the fluid drainage rate on the set-up screens but may be configured that the user can set up the fluid drainage rate on the set-up screens. If it is decided in step S6 that fluid drainage is performed for a fluid drainage time of more than a specific ratio based on the calculated fluid drainage time (min), for example, for a fluid drainage time being twice or more the calculated fluid drainage time (min), the process goes on to step S7-2. In step S4-2, an alarm indicating defective fluid drainage is displayed on the displaying portion 23 together with the corresponding voice guide. The fluid drainage time (min) is determined by adding a preparation time (min) until start of fluid drainage to a value of fluid drainage amount (mL)/fluid drainage rate (mL/min). The preparation time (min) is set, for example, to about 2 min. If the amount of drained amount is returned to a predetermined amount of drained amount (mL) or fluid drainage is performed at a predetermined fluid drainage rate, the process goes on to step S7-1 in which a normal image indicated by a character is displayed on the displaying portion 23. It is to be noted that the calculation of fluid drainage time (min) may be performed together with the calculation of the fluid infusion time in step S2.

A program for a detection mode of defective fluid infusion and a program for a detection mode of defective fluid drainage are stored in either the storing unit 152 or the storage medium 170a, and are read out by the storage medium reading portion 170 and controlled by the CPU 150.

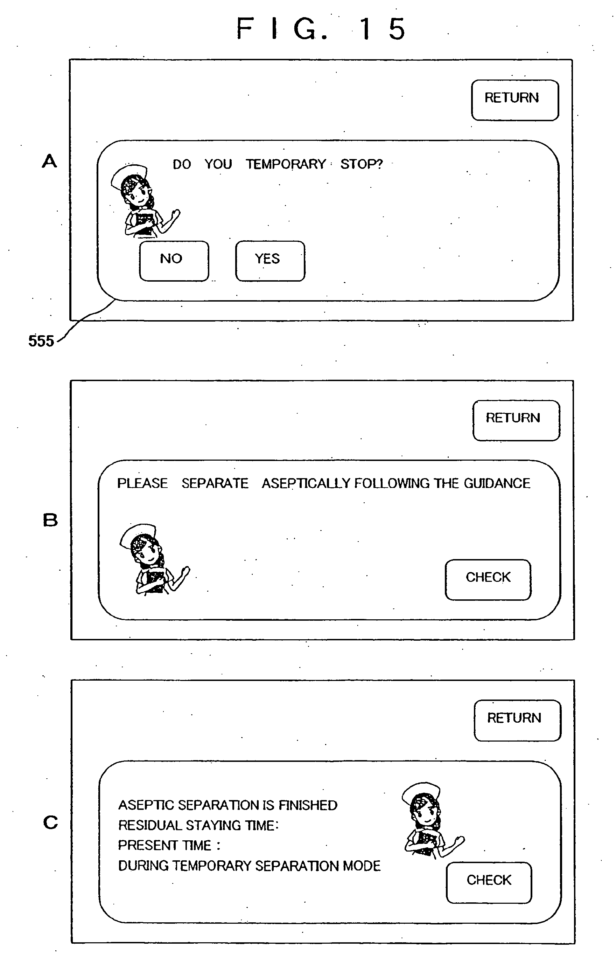

FIGS. 15A to 15C show an example of a guide screen appearing on the displaying portion 23 at the time of temporarily separating the dialysis catheter 7 by an aseptic separating/joining apparatus. When the touch key "ALTER" on the screen 503 in FIG. 8 is depressed, the screen 503 is changed to a screen 555 in FIG. 15A (step S1). On the screen 555, an image indicating whether or not a "temporary stoppage (temporary separation) mode is selected is displayed. If it is intended to perform the temporary stoppage, a touch key "YES" may be depressed, and in this case, the screen is changed to a screen shown in FIG. 15B (step S2), on which a procedure of aseptically separating the dialysis catheter (catheter tube) (see FIGS. 2 and 6) implanted in the peritoneal cavity from the peritoneal dialysis fluid circuit is displayed together with the corresponding voice guide (step S3). If the dialysis catheter has been separated in accordance with the procedure, the screen is changed to a screen shown in FIG. 15C, on which a message "The aseptic separation (cutting) has been ended" is displayed together with the corresponding voice guide, and at this time, by depressing a touch key "CHECKING", a message "During temporary separation mode" is displayed together with messages "Residual staying time" and "Present time" (step S4). The messages "Residual staying time", "Present time", and "During temporary separation mode" can be checked on the screen 1023 of the portable terminal 1000. When the residual staying time exceeds a specific time (min), an alarm is generated in the form of a buzzer, voice sound, or the like. A program for the temporary separation mode may be stored in the storage medium 170a such as a flexible disk or a CD-ROM and read out by the storage medium reading portion 170, or may be previously stored in the storing unit 152.

## Claims

1. A peritoneal dialysis apparatus (1) including at least one dialysis fluid container (4, 5) filled with a dialysis fluid, a dialysis fluid circuit (3) containing at least one drained fluid container (6) for recovering the dialysis fluid, fluid delivery means (15) for delivering the dialysis fluid with said dialysis fluid container (4, 5) as a start point or said drained fluid container (6) as an end point, input means (23) for inputting dialysis conditions including an amount of infusion fluid (mL) and a fluid drainage rate (mL/min), and display means (23) for displaying the inputted dialysis conditions, wherein dialysis is performed by delivering the dialysis fluid to a patient by said delivery means and recovering the drained fluid, the apparatus including means for generating an alarm indicating defective fluid drainage if it is decided that fluid drainage is defective;
**characterized in that** the apparatus further includes:
means for calculating an amount of drainage fluid (mL) on the basis of the inputted dialysis conditions, and for calculating a fluid drainage time (min) on the basis of the calculated amount of drainage fluid (mL) and the fluid drainage rate (mL/min); and
means for deciding whether or not fluid drainage is performed for a fluid drainage time (min) which is more than a specific ratio of the calculated fluid drainage time (min).

2. A peritoneal dialysis apparatus (1) according to claim 1, wherein the fluid drainage time (min) is obtained by adding a preparation time until the start of fluid drainage to a value of the amount of drainage fluid (mL)/the fluid drainage rate (mL/min).

## Patentansprüche

1. Peritonealdialysegerät (1), das zumindest einen mit einem Dialysefluid gefüllten Dialysefluidbehälter (4, 5) umfasst, wobei ein Dialysefluidkreislauf (3) zumindest einen Ableitfluidbehälter (6) zur Wiederaufbereitung des Dialysefluids, ein Fluidzufuhrmittel (15) zum Zuführen des Dialysefluids, mit dem Dialysefluidbehälter (4, 5) als Ausgangspunkt bzw. dem Ableitfluidbehälter (6) als Endpunkt, ein Eingabemittel (23) zum Eingeben von Dialysebedingungen, einschließlich der Menge an Infusionsfluid (ml) und der Fluidableitgeschwindigkeit (ml/min) sowie ein Anzeigemittel (23) zum Anzeigen der eingegebenen Dialysebedingungen enthält, wobei die Dialyse durch Abgabe des Dialysefluids an einen Patienten durch das Zufuhrmittel und durch Wiederaufbereitung des Ableitfluids durchgeführt wird, wobei das Gerät ein Mittel zum Auslösen eines Alarms umfasst, der eine fehlerhafte Fluidableitung anzeigt, wenn entschieden wird, dass die Fluidableitung fehlerhaft ist;
**dadurch gekennzeichnet, dass** das Gerät ferner Folgendes umfasst:
ein Mittel zur Berechnung einer Fluidableitmenge (ml) auf Basis der eingegebenen Dialysebedingungen und zur Berechnung einer Fluidableitzeit (min) auf Basis der ermittelten Menge an Ableitfluid (ml) und der Fluidableitgeschwindigkeit (ml/min); und
ein Mittel, um zu entscheiden, ob eine Fluidableitung für eine Fluidableitzeit (min) durchgeführt wird, die mehr als ein spezifisches Verhältnis der berechneten Fluidableitzeit (min) beträgt.

2. Peritonealdialysegerät (1) nach Anspruch 1, worin die Fluidableitzeit (min) durch Addieren einer Vorbereitungszeit bis zum Beginn der Fluidableitung zu einem Wert der Menge an Ableitfluid (ml)/Fluidableitgeschwindigkeit (ml/min) erhalten wird.

## Revendications

1. Appareil de dialyse péritonéale (1), comprenant au moins un récipient de fluide de dialyse (4, 5) rempli d'un fluide de dialyse, un circuit de fluide de dialyse (3) contenant au moins un récipient de fluide évacué (6) pour récupérer le fluide de dialyse, des moyens de distribution de fluide (15) pour distribuer le fluide de dialyse avec ledit récipient de fluide de dialyse (4, 5) comme point initial ou avec ledit récipient de fluide évacué (6) comme point final, des moyens d'entrée (23) pour entrer des conditions de dialyse comprenant une quantité de fluide de perfusion (mL) et un débit d'évacuation de fluide (mL/min), et des moyens d'affichage (23) pour afficher les conditions de dialyse entrées, dans lequel la dialyse est effectuée en distribuant le fluide de dialyse à un patient par lesdits moyens de distribution et en récupérant le fluide évacué, l'appareil comprenant des moyens pour générer une alarme indiquant une évacuation de fluide défectueuse s'il est décidé que l'évacuation de fluide est défectueuse ;
**caractérisé en ce que** l'appareil comprend en outre:
des moyens pour calculer une quantité de fluide d'évacuation (mL) sur la base des conditions de dialyse entrées, et pour calculer un temps d'évacuation de fluide (min) sur la base de la quantité calculée de fluide d'évacuation (mL) et du débit d'évacuation de fluide (mL/min) ; et
des moyens pour décider si une évacuation de fluide est effectuée ou non pendant un temps d'évacuation de fluide (min) qui est supérieur à un taux spécifique du temps d'évacuation de fluide calculé (min).

2. Appareil de dialyse péritonéale (1) selon la revendication 1, dans lequel le temps d'évacuation de fluide (min) est obtenu en ajoutant un temps de préparation jusqu'au début de l'évacuation de fluide à une valeur de la quantité de fluide d'évacuation (mL)/ débit d'évacuation de fluide (mL/min).
